(19) Europäisches Patentamt
European Patent Office
Office européen des brevets

(11) EP 1 491 896 A1

(12) **EUROPEAN PATENT APPLICATION**
published in accordance with Art. 158(3) EPC

(43) Date of publication:
**29.12.2004 Bulletin 2004/53**

(21) Application number: **03715565.2**

(22) Date of filing: **28.03.2003**

(51) Int Cl.⁷: **G01N 33/68**, G01N 33/53, G01N 33/15

(86) International application number:
**PCT/JP2003/003924**

(87) International publication number:
**WO 2003/083486 (09.10.2003 Gazette 2003/41)**

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(30) Priority: **29.03.2002 JP 2002093688**

(71) Applicant: **Dainippon Pharmaceutical Co., Ltd.**
**Osaka-shi, Osaka 541-8524 (JP)**

(72) Inventors:
- **KITAYAMA, Hitoshi**
  **Toyonaka-shi, Osaka 560-0002 (JP)**
- **OHKARU, Yasuhiko**
  **Sennan-gun, Osaka 599-0301 (JP)**

(74) Representative: **von Kreisler, Alek, Dipl.-Chem.**
**Patentanwälte**
**von Kreisler-Selting-Werner**
**Postfach 10 22 41**
**50462 Köln (DE)**

(54) **METHOD OF JUDGING CARDIOTOXICITY OF ANTHRACYCLINE-TYPE ANTICANCER CHEMICAL THERAPEUTIC BY DETECTING HUMAN H-FABP AND REAGENT THEREFOR**

(57) The present invention provides a method of determining toxicity to the heart of an anthracycline-type anticancer chemotherapeutic agent such as adriamycin etc., which comprises detecting human H-FABP (Human Heart-type Fatty Acid-Binding Protein) in the blood separated from human, a reagent therefor, a kit therefor and the like.

EP 1 491 896 A1

## Description

### Technical Field

This invention relates to a method of determining toxicity to the heart of anthracycline-type anticancer chemotherapeutic agents, of which particularly Doxorubicin hydrochloride (in the present specification, "adriamycin" which is a popular name of the present compound is used) (hereinafter sometimes to be referred to as "cardiotoxicity"), a reagent for the determination and the like.

### Background Art

The anthracycline-type anticancer chemotherapeutic agent is a glycoside consisting of an aglycon part comprising a 4-membered ring quinone structure as a basic skeleton and saccharides mainly consisting of an amino sugar. As a pharmaceutical agent of this type, for example, adriamycin, daunorubicin hydrochloride, epirubicin hydrochloride, idarubicin hydrochloride, pirarubicin hydrochloride, aclarubicin hydrochloride and the like having the following structures can be mentioned.

### Examples of anthracycline-type anticancer chemotherapeutic agents

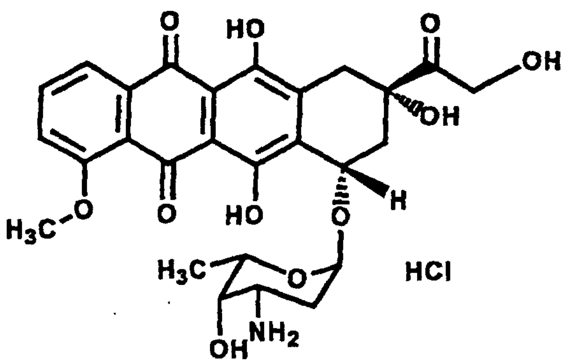

adriamycin

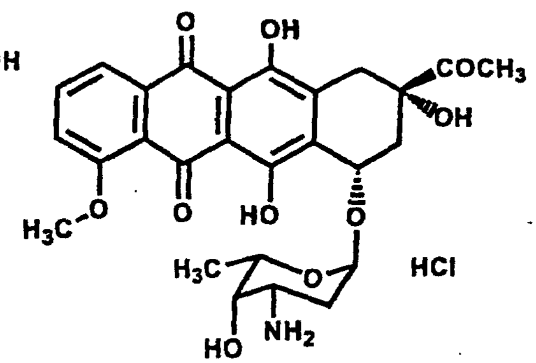

daunorubicin hydrochloride

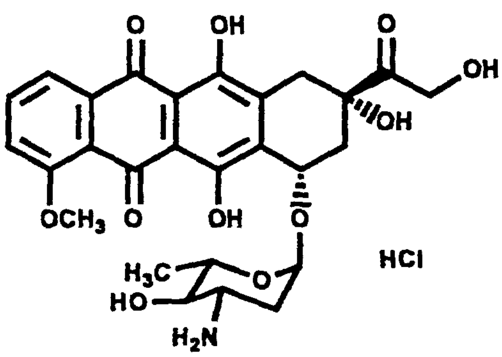

epirubicin hydrochloride

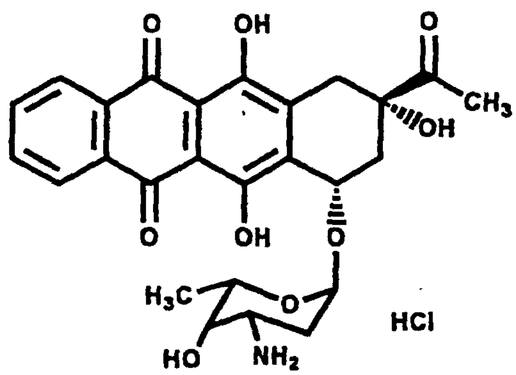

idarubicin hydrochloride

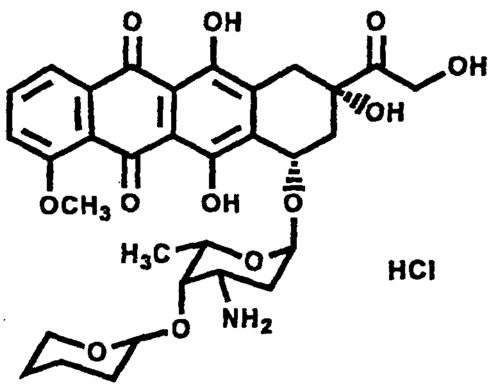

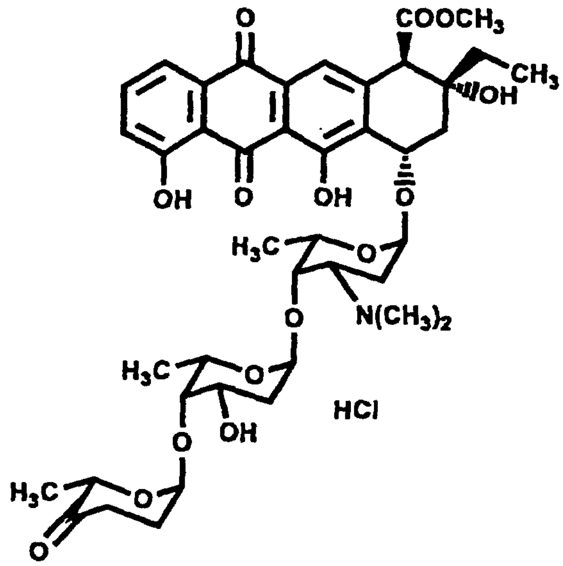

pirarubicin hydrochloride    aclarubicin hydrochloride

For cancer treatment, prolonged administration of anthracycline-type anticancer chemotherapeutic agents is generally employed. While anthracycline-type anticancer chemotherapeutic agents have a wide range of anticancer spectrum, they are known to show cardiotoxicity as a common side effect due to myocardial injury action.

As a method of determining toxicity to the heart of an anthracycline-type anticancer chemotherapeutic agent, an electrocardiogram analysis, a blood biochemical test comprising measurement of Creatine Kinase (CK) in blood, an echocardiogram analysis and the like, which are general tests of cardiac function, are conventionally known and performed. However, since electrocardiogram analysis and echocardiogram analysis do not specifically detect cardiotoxicity of anthracycline-type anticancer chemotherapeutic agents, they do not have sufficient sensitivity to pick up the initial stage of the onset of toxicity of the agents, and can detect only the advanced cardiotoxicity. In addition, only a small amount of creatine kinase flows (escapes) into the blood due to the cardiotoxicity induced by anthracycline-type anticancer chemotherapeutic agents and creatine kinase requires a long time before escape, and therefore, a problem in clinical situation has existed in that cardiotoxicity of anthracycline-type anticancer chemotherapeutic agents is not precisely reflected.

On the other hand, as a myocardial injury marker that detects acute myocardial infarction and the like, Troponin T (TnT), Myosin Light Chain I (MLC-I), human H-FABP (Human Heart-type Fatty Acid-Binding Protein) and the like have been known.

Human H-FABP is abundant in myocardial cytoplasm, capable of binding with fatty acid and is considered to be involved in the intracellular transport of fatty acid. Human H-FABP is reported to be a protein consisting of 132 amino acids and has a molecular weight of 14,768 (Biochem. J. (1988) 252 191-198).

With regard to Human H-FABP, JP-A-4-31762 discloses that this protein is useful as a marker of acute myocardial infarction. However, the above-mentioned publication does not at all refer to the relationship between the cardiotoxicity of anthracycline-type anticancer chemotherapeutic agents and Human H-FABP.

Therefore, the relationship between human H-FABP and the cardiotoxicity of anthracycline-type anticancer chemotherapeutic agent has not been known at all to the present day.

## Disclosure Of The Invention

An object of the present invention is to provide a novel method of determining the toxicity to the heart of anthracycline-type anticancer chemotherapeutic agents such as adriamycin and the like and a reagent for the determination.

To solve these problems, the present inventors have compared and studied the levels of Myosin Light Chain I, Troponin T and human H-FABP in the blood of cancer patients who have been administered with adriamycin, which is a representative anthracycline-type anticancer chemotherapeutic agent, and with whom the expression of cardiotoxicity has been confirmed by electrocardiogram analysis and echocardiogram analysis, and found that, while all these

markers belong to myocardial injury markers to detect acute myocardial infarction and the like, only the level of human H-FABP has increased to not less than the cut-off value of acute myocardial infarction unique to each marker, which resulted in the completion of the present invention.

Therefore, the present invention provides the following.

[1] A method of determining toxicity to the heart of an anthracycline-type anticancer chemotherapeutic agent, which comprises detecting human H-FABP in the blood separated from human.
[2] The method of [1] above, wherein the detection of human H-FABP is performed by an immunochemical method using an antibody that recognizes human H-FABP.
[3] The method of [2] above, wherein the immunochemical method is an enzyme immunochemical method, a latex agglutination assay or an immunochromatographic assay.
[4] The method of [2] above, wherein the antibody is a monoclonal antibody.
[5] The method of [1] above, wherein the anthracycline-type anticancer chemotherapeutic agent is adriamycin or daunorubicin hydrochloride.
[6] A reagent for determining toxicity to the heart of an anthracycline-type anticancer chemotherapeutic agent, which is used for performing the method of any of [1] to [5] above.
[7] A reagent for determining toxicity to the heart of an anthracycline-type anticancer chemotherapeutic agent, which comprises an antibody that recognizes human H-FABP.
[8] The reagent of [7] above, wherein the antibody is a monoclonal antibody.
[9] The reagent of [7] above, wherein the anthracycline-type anticancer chemotherapeutic agent is adriamycin or daunorubicin hydrochloride.
[10] A commercial package, comprising the reagent of any of [7] to [9] above, and a written matter associated therewith, the written matter stating that said reagent can or should be used for determining toxicity to the heart of an anthracycline-type anticancer chemotherapeutic agent.
[11] A kit for determining toxicity to the heart of an anthracycline-type anticancer chemotherapeutic agent, which comprises an antibody that recognizes human H-FABP.
[12] The kit of [11] above, wherein the antibody is a monoclonal antibody.
[13] The kit of [11] above, wherein the anthracycline-type anticancer chemotherapeutic agent is adriamycin or daunorubicin hydrochloride.
[14] Use of an antibody that recognizes human H-FABP for determining toxicity to the heart of an anthracycline-type anticancer chemotherapeutic agent.
[15] The use of [14] above, which comprises detecting human H-FABP in the blood separated from human.
[16] The use of [15] above, wherein the detection of human H-FABP is performed by an enzyme immunochemical method, a latex agglutination assay or an immunochromatographic assay.
[17] The use of [14] above, wherein the antibody is a monoclonal antibody.
[18] The use of [14] above, wherein the anthracycline-type anticancer chemotherapeutic agent is adriamycin or daunorubicin hydrochloride.

The present invention provides a method of determining toxicity to the heart of an anthracycline-type anticancer chemotherapeutic agent, which comprises detecting human H-FABP in the blood separated from human. According to this determination method, determination of the cardiotoxicity of the patients under medication with anthracycline-type anticancer chemotherapeutic agents such as adriamycin and the like becomes possible. To be specific, by drawing blood from the above-mentioned patients, comparing the level of Human H-FABP contained therein with that of human H-FABP contained in the blood of healthy volunteers, and further by comparing with cut-off value for the determination of acute myocardial infarction, whether or not the cardiotoxicity has been expressed, and when it has been expressed, the level of the toxicity can be determined.

In addition, the present invention also provides a reagent for determining toxicity to the heart of an anthracycline-type anticancer chemotherapeutic agent, which is used for performing the determination method of the present invention, and the like. This reagent for the determination and the like are directly used for practicing the determination method of the present invention, and achieve the same object as the determination method of the present invention.

**Best Mode For Carrying Out The Invention**

The present invention relates to a method of determining toxicity to the heart of an anthracycline-type anticancer chemotherapeutic agent, which comprises detecting human H-FABP in the blood separated from human.

The cardiotoxicity to be the target of determination in the present invention arises from the side effect of an anthracycline-type anticancer chemotherapeutic agent. Examples of the above-mentioned chemotherapeutic agent include conventionally known various agents such as adriamycin, daunorubicin hydrochloride, epirubicin hydrochloride,

idarubicin hydrochloride, pirarubicin hydrochloride, aclarubicin hydrochloride and the like. The determination method of the present invention is particularly preferable for determining the cardiotoxicity due to adriamycin or daunorubicin hydrochloride, from among these anthracycline-type anticancer chemotherapeutic agents.

In the present invention, by the "determination of toxicity" is meant not only assumption of the presence or absence of expressed toxicity, but also assumption of the level of toxicity when toxicity is present.

While the determination of toxicity may depend exclusively on the detection of Human H-FABP, other known methods for the detection of abnormality in the heart, such as electrocardiogram analysis, echocardiogram analysis and the like may be used in combination to determine toxicity. By combining plural test methods, the toxicity can be determined more accurately.

A method of detecting human H-FABP in the blood separated from human in the present invention is not particularly limited, and may be any method; for example, conventionally known methods for quantification, semi-quantification or qualitative analysis, such as immunochemical method, fatty acid binding activity measurement method, various chromatographies (e.g., HPLC and the like), and the like.

Of these, the detection is particularly preferably performed by a method aiming at quantification of human H-FABP because the level and progress of cardiotoxicity can be determined, and the detection is particularly preferably performed by an immunochemical method utilizing an antibody recognizing human H-FABP (hereinafter sometimes to be referred to as an "anti-human H-FABP antibody").

When human H-FABP is detected by an immunochemical method, an immunochemical method using any of a monoclonal antibody and a polyclonal antibody as an anti-human H-FABP antibody can be preferably used. In view of stable supply of antibody and specificity to human H-FABP, an immunochemical method using a monoclonal antibody is preferably used. Anti-human H-FABP antibody itself is known, and can be produced according to the production method described in JP-A-4-31762 or a method analogous thereto.

The immunochemical method is not particularly limited, and, for example, a conventionally known Enzyme Immunoassay (EIA), Latex Agglutination Assay, Immunochromatographic Assay, Radio Immunoassay (RIA), Fluorescence Immunoassay (FIA), Luminescence Immunoassay, Spin Immunoassay and the like can be mentioned. Of these, EIA, Latex Agglutination Assay and Immunochromatographic Assay are preferable. In EIA, sandwich Enzyme-Linked Immunosorbent Assay (sandwich ELISA) using two kinds of antibodies, particularly monoclonal antibodies, is particularly preferable in view of specificity to antigen (human H-FABP) and easiness of detection operation.

When the above-mentioned human H-FABP is to be detected by sandwich ELISA, for example, according to the description in Journal of Immunological Methods 178 (1995) 99-111 and the like, human H-FABP is inserted (sandwiched) between two kinds of antibodies that recognize different epitopes of human H-FABP, i.e., solid phase antibody and enzyme-labeled antibody, and the enzyme level of the labeled antibody that was bound with human H-FABP is determined, whereby quantitative detection is performed.

The Latex Agglutination Assay is an immunochemical method utilizing an agglutination reaction between an antibody sensitized Latex particle and an antigen. When the above-mentioned human H-FABP is to be detected by the Latex Agglutination Assay, quantitative detection is performed by measuring the level of agglutination.

In the Immunochromatographic Assay, all immunochemical reaction systems are retained on a sheet carrier, wherein the operation is completed only by the addition (dropwise addition) of blood. In other words, when the blood is added to the carrier, human H-FABP in the blood is bonded to an anti-human H-FABP antibody labeled with gold colloid and the like, and this bonded form is chromatographically developed on the carrier and captured at a particular site (determination region) by a different solid phased anti-human H-FABP antibody. When the above-mentioned human H-FABP is to be detected by this Immunochromatographic Assay, because the bonded form is captured as mentioned above, the accumulated sample materials can be detected by visual observation. Since this method does not require a special measuring instrument for practicing, it is an advantageous method when a rapid treatment becomes necessary depending on the determination results.

As measurement methods besides the above-mentioned that utilizes specificity of an anti H-FABP antibody, nephelometry comprising measurement of turbidity associated with an antigen-antibody complex formation, an enzyme sensor electrode method comprising detection of electrical potential change due to antigen bond, which utilizes an antibody solid phase membrane electrode, immunoelectrophoresis, western blotting and the like can be mentioned. Any of these methods suffices for detection of human H-FABP.

As the blood for the detection of human H-FABP in the present invention, any blood can be used as long as it is isolated from human, and may be any of whole blood, blood serum and blood plasma. The above-mentioned blood can be obtained appropriately by a treatment according to a conventional method per se.

The determination of toxicity to the heart of an anthracycline-type anticancer chemotherapeutic agent such as adriamycin and the like can be performed by comparing the human H-FABP level in the blood detected in this manner with the human H-FABP level in the blood of healthy volunteers, and by comparing with a cut-off value of myocardiopathy (e.g., acute myocardial infarction).

For example, according to Okamoto et al., Clinical Chemistry and Laboratory Medicine 2000 38(3) 231-238,

average value of the human H-FABP level in the blood (blood serum) of healthy volunteer is 2.8 ng/mL (upper limit being 5.3 ng/mL), and the cut-off value of acute myocardial infarction, or the level that showed the highest diagnostic efficiency (value satisfying high true positive rate (diagnostic sensitivity) and high true negative rate (diagnostic specificity)) is set to 6.2 ng/mL.

Therefore, when human H-FABP level in the blood of a cancer patient under medication with an anthracycline-type anticancer chemotherapeutic agent exceeds the upper limit of human H-FABP level in the blood of healthy volunteer, 5.3 ng/mL, the presence of suspect of expression of cardiotoxicity can be determined, and when it increases to the cut-off value of acute myocardial infarction, i.e., not less than 6.2 ng/mL, the possibility of expression of cardiotoxicity can be determined to be extremely high.

The determination results thus obtained are useful for a physician to decide, for a cancer patient under medication with an anthracycline-type anticancer chemotherapeutic agent, if (1) the administration of the same agent is to be continued, (2) the administration is to be stopped (the kind (type) of anticancer agent is changed) or (3) the dose is to be increased or decreased, and the like, and for a patient for whom administration of an anthracycline-type anticancer chemotherapeutic agent was once stopped due to the expression of cardiotoxicity, it is useful for determining if (4) administration of this agent is to be resumed.

To be specific, during the administration period of adriamycin, for example, blood is taken from a patient at a frequency of at least once a month and human H-FABP level is measured using the obtained blood as a sample. In this case, when a human H-FABP level shows not less than 6.2 ng/mL, which is a cut-off value of acute myocardial infarction, administration of adriamycin is quickly stopped, and an anticancer agent of a different kind (type) is employed. Since human H-FABP level is considered to also reflect the level of cardiotoxicity due to adriamycin, when human H-FABP exceeds 6.2 ng/mL and the value is at a high level, the injury the cardiac muscle suffered from is considered to be large. Thus, it is determined that the administration of the agent is to be stopped and a quick protective measure of cardiac muscle needs to be taken.

In contrast, when human H-FABP level is less than 6.2 ng/mL, particularly less than 5.3 ng/mL, it is determined that administration of adriamycin can be continued without a problem.

The present invention also relates to a reagent for determining toxicity to the heart of an anthracycline-type anticancer chemotherapeutic agent, which is used to practice the above-mentioned determination method. Thus, the reagent for determination of the present invention is directly used for the practice of the aforementioned determination method of the present invention, and achieves the same object as the determination method of toxicity.

In addition, the present invention provides a reagent and a kit for determining toxicity to the heart of an anthracycline-type anticancer chemotherapeutic agent at least containing an antibody recognizing Human H-FABP (anti-human H-FABP antibody). Such reagent and a kit for determination can be preferably used for, among the aforementioned determination methods of the present invention, detection of human H-FABP by an immunochemical method in blood separated from human.

The anti-human H-FABP antibody can be produced according to a known method, such as the method described in, for example, the aforementioned JP-A-4-31762, and can be applied to an immunochemical method in a free state, a labeled state or a solidified state. The anti-human H-FABP antibody may be a polyclonal antibody or a monoclonal antibody, but a monoclonal antibody is preferable because specificity and uniformity of antibody are high. A polyclonal antibody can be produced by immunizing an animal such as mouse, rat, rabbit and the like with human H-FABP together with a suitable adjuvant, taking blood and processing the blood by known treatments. In addition, a monoclonal antibody can be produced by harvesting spleen cells of an animal immunized in this way, subjecting the cells to cell fusion with myeloma cells, and to antibody-producing cells screening and cloning etc. by the method of Milstein et al., thereby establishing an anti-human H-FABP antibody-producing cell line, and culturing the cell line. Here, human H-FABP to be used as an immunizing antigen is not necessarily a human cardiac muscle organ derived, natural H-FABP, and may be a recombinant human H-FABP obtained by genetic engineering technique or any material having the same effect (fragment).

When the thus-obtained anti-human H-FABP antibody is applied to sandwich ELISA, this antibody is produced in the form of a solid phased anti-human H-FABP antibody or an enzyme-labeled anti-human H-FABP antibody.

A solid phased anti-human H-FABP antibody can be produced by binding an antibody obtained as mentioned above to a solid phase (e.g., microplate well or plastic beads). Binding to a solid phase can be generally performed by dissolving an antibody in a suitable buffer solution such as citrate buffer solution and the like, and bringing a solid phase surface in contact with an antibody solution for a suitable time (1-2 days). Then, phosphate buffer containing Bovine Serum Albumin (BSA), bovine milk protein and the like is brought into contact with the solid phase to coat a part on the surface of the solid phase that was not coated with the antibody with the aforementioned BSA and the like.

An enzyme-labeled anti-human H-FABP antibody can be produced by binding (labeling) an anti-human H-FABP antibody, which recognizes an epitope different from the above-mentioned solid phased antibody, with an enzyme. As the enzyme to label the antibody, alkaline phosphatase, glucose oxidase, peroxidase, β-galactosidase and the like can be mentioned. The binding between these enzymes and anti-human H-FABP antibody can be performed by a method known per se, such as glutaraldehyde method, maleimide method and the like. In addition, a method using an avidin-biotin reaction (method comprising reacting an antibody labeled with biotin instead of an enzyme with human H-FABP in blood and then binding with an enzyme-labeled streptavidin) may be employed.

In sandwich ELISA, enzyme substrate, washing solution, reaction-stopping solution, substrate dissolution solution, standard antigen (human H-FABP) and the like are used as necessary, besides anti-human H-FABP antibody. The present invention may be realized in the form of a kit (kit for determining toxicity to the heart of an anthracycline-type anticancer chemotherapeutic agent) containing these as components, besides the above-mentioned anti-human H-FABP antibody.

As the enzyme substrate, one suitable for a selected labeled enzyme is used. For example, when alkaline phosphatase is selected as an enzyme, p-nitrophenylphosphate (PNPP) and the like are mentioned, and as a color developing agent in this case, o-phenylenediamine (OPD), tetramethylbenzidine (TMB) and the like are used. As the washing solution, reaction-stopping solution and substrate dissolution solution, conventionally known ones are appropriately used without any particular limitation, depending on the selected labeled enzyme.

A specific production method of the reagent for determination of the present invention based on sandwich ELISA is described in Ohkaru et al., Journal of Immunological Methods 178 (1995) 99-111. In addition, a blood serum human H-FABP measurement kit ("MARKIT (registered trademark) M H-FABP") based on sandwich ELISA as a measurement principle has been available from DAINIPPON PHARMACEUTICAL CO., LTD., and this kit can be used as a determination kit relating to the present invention.

In addition, a reagent that detects human H-FABP by o Latex Agglutination Assay is also known and its production method is also specifically described in, for example, Markus Robers et al., Clinical Chemistry 44, No. 7, (1998) 1564-1567 and the above-mentioned JP-A-4-31762.

It is also possible to apply an Immunochromatographic Assay to the reagent for determination of the present invention. Specific production method of the reagent for determination of the present invention by Immunochromatographic Assay is described in Watanabe et al., Clinical Biochemistry 34 (2001) 257-263. Furthermore, a whole blood human H-FABP detection reagent ("RAPICHECK (registered trademark) H-FABP") based on Immunochromatographic Assay as a detection principle has been available from DAINIPPON PHARMACEUTICAL CO., LTD., and this can be used as a reagent for determination relating to the present invention.

The present invention is explained in detail by referring to Examples and Reference Examples, which are not to be construed as limitative.

**Example 1**

The blood from a cancer patient administered with adriamycin was taken three times during 5 months of adriamycin administration (second blood sampling was about 2.5 months after the first sampling, third blood sampling was 20 days after the second sampling) and blood serum was obtained by a conventional method. Using the obtained blood serum as a sample, the level of human H-FABP, Myosin Light Chain I and Troponin T was measured.

Human H-FABP was measured using a blood serum human H-FABP measurement kit "MARKIT (registered trademark) M H-FABP" (manufactured by DAINIPPON PHARMACEUTICAL CO., LTD.) based on sandwich ELISA using two kinds of specific monoclonal antibodies as a measurement principle.

That is, a 1:1 volume mixture (100 μL) of a diluting buffer solution (composition: 0.2% BSA - 0.9% NaCl - 0.1 mol/L potassium phosphate buffer, pH 7.0) and a blood serum sample were dispensed to a microplate well (antibody bonded well), in which one kind of anti-human H-FABP monoclonal antibody had been solid phased, and the reaction was carried out at room temperature (25°C) for 30 min (first antigen-antibody reaction). After the reaction, the antibody bonded well was washed 3 times with 300 μL of washing solution, then horseradish peroxidase-labeled anti-human H-FABP monoclonal antibody (100 μL) was dispensed thereto and the reaction was carried out at room temperature for 30 min (second antigen-antibody reaction). After washing in the same manner as above, an enzyme substrate (100 μL, hydrogen peroxide-containing OPD) was added to start the enzyme reaction, which was carried out at room temperature for 15 min. A reaction-stopping solution (100 μL, 0.9 mol/L sulfuric acid) was added to stop the reaction. As a control, the above-mentioned diluting buffer solution and each standard solution prepared to make the final concentration of human H-FABP 0 ng/mL, 5 ng/mL, 10 ng/mL, 25 ng/mL, 50 ng/mL, 100 ng/mL, 250 ng/mL were mixed at a volume ratio of 1:1 and processed in the same manner as above.

The level of color development of each well was determined by measuring an absorbance at 492 nm, and comparing with a standard curve drawn from the results of a standard solution containing human H-FABP at each concentration employed for the control, whereby the human H-FABP levels in the blood serum samples were read. In addition, the cut-off value of acute myocardial infarction for the above-mentioned blood serum human H-FABP measurement kit was set for 6.2 ng/mL.

For the measurement of Myosin Light Chain I, sandwich RIA was used and for the measurement of Troponin T, sandwich ELISA was used. The cut-off values of acute myocardial infarction in this case were set to 2.5 ng/mL for

Myosin Light Chain I, and 0.1 ng/mL for Troponin T.

o The state of the cardiac function was diagnosed by electrocardiogram analysis, echocardiogram analysis and measurement of creatine kinase in blood.

By the echocardiogram analysis, the heart pump function was evaluated based on the ejection fraction (EF) of the heart as calculated by the following formula:

$$\text{Ejection fraction (\%)} = \{(\text{left ventricular relaxation terminal diameter}^2 - \text{left ventricular contraction terminal diameter}^2) / \text{left ventricular relaxation terminal diameter}^2\} \times 100$$

The ejection fraction of healthy volunteers is known to be not less than 60%.

The normal range of blood creatine kinase in healthy volunteers is known to be 25 U/L-180 U/L for male, and 20 U/L-150 U/L for female.

Table 1 shows the results of the ejection fraction of the heart, the measurement of blood creatine kinase and the above-mentioned three kinds of each marker.

Table 1

| Blood sampling | EF (%) | Blood concentration | | | |
|---|---|---|---|---|---|
| | | Creatine kinase (U/L) | Human H-FABP (ng/mL) | Myosin Light Chain I (ng/mL) | Troponin T (ng/mL) |
| First time | 53 | 37 | 6.8 | < 1.0 | < 0.01 |
| Second time | - | 36 | 9.0 | < 1.0 | 0.01 |
| Third time | 37 | 33 | 19.6 | <1.0 | 0.41 |

-: no data

ejection fraction of healthy volunteer: not less than 60% creatine kinase level of healthy volunteer: 25-180 U/L (male), 20-150 U/L (female)

cut-off value of acute myocardial infarction in this measurement;

Human H-FABP: 6.2 ng/mL, Myosin Light Chain I: 2.5 ng/mL, Troponin T: 0.1 ng/mL

While not described in Table 1, in the electrocardiogram analysis of this patient, extension of QTc at about 2 weeks before the first blood sampling was observed and T wave decreased at about 1 week before the third blood sampling. In addition, sinus tachycardia and supraventricular extrasystole were also observed then.

As shown in the above-mentioned Table 1, the ejection fraction was lower than the value of healthy volunteer (not less than 60%) in the first blood sampling, and the toxicity to the heart due to adriamycin was considered to have been expressed. However, the level of creatine kinase was within the normal range, and the toxicity could not be detected. At this time point, of the three kinds of markers, only the level of human H-FABP exceeded the above-mentioned cut-off value (6.2 ng/mL), and the levels of Myosin Light Chain I and Troponin T were not more than the measurement limits.

In the second blood sampling, the condition of the heart worsened as compared to the first blood sampling and stronger toxicity is considered to have expressed, in view of the findings from the electrocardiogram analysis and the like. However, from the level of creatine kinase, the toxicity could not be detected as in the previous time. At this time point, the human H-FABP level exceeded the above-mentioned cut-off value and increased further. In contrast, the level of Myosin Light Chain I was not more than the measurement limit, and the level of Troponin T was not more than the cut-off value.

In the third blood sampling, the ejection fraction decreased to 37%, and the condition of the heart was worse than in the first and the second blood samplings, and still stronger toxicity is considered to have expressed. However, from the level of creatine kinase, the toxicity still could not be detected. At this time point, the human H-FABP level far exceeded the above-mentioned cut-off value. In contrast, the level of Myosin Light Chain I was not more than the measurement limit, and the level of Troponin T first exceeded the above-mentioned cut-off value.

**Example 2**

Blood human H-FABP and the like of cancer patient who was administered with daunorubicin hydrochloride and diagnosed with cardiac failure due to the expression of generalized edema were detected in the same manner as in the method described in Example 1. The results thereof are shown in the following Table 2. The second blood sampling in the Table was performed one month after the first blood sampling.

Table 2

| Blood sampling | Blood concentration | | | |
|---|---|---|---|---|
| | Creatine kinase (U/L) | Human H-FABP (ng/mL) | Myosin Light Chain I (ng/mL) | Troponin T (ng/mL) |
| First time | 11 | 7.6 | < 1.0 | < 0.01 |
| Second time | 7 | 6.9 | 1.4 | < 0.01 |

creatine kinase level of healthy volunteer: 25-180 U/L (male), 20-150 U/L (female)
cut-off value of acute myocardial infarction in this measurement;
Human H-FABP: 6.2 ng/mL, Myosin Light Chain I: 2.5 ng/mL, Troponin T: 0.1 ng/mL

The above-mentioned Table 2 reveals that, in the first and the second blood samplings, human H-FABP alone exceeded the cut-off value (6.2 ng/mL) of acute myocardial infarction, and other myocardial injury markers, i.e., Myosin Light Chain I and Troponin T, were within the normal ranges. As shown in Table 2, creatine kinase did not exceed the level of healthy volunteer.

As shown in Table 1 and Table 2 above, of the three kinds of myocardial injury markers, only human H-FABP sensitively detected expression of the cardiotoxicity due to adriamycin or daunorubicin hydrochloride.

Therefore, blood human H-FABP is a useful marker to predict the presence and the level of cardiotoxicity caused by an anthracycline-type anticancer chemotherapeutic agent such as adriamycin and the like.

**Industrial Applicability**

According to the present invention, namely, a method of determining cardiotoxicity caused by an anthracycline-type anticancer chemotherapeutic agent, which comprises detecting blood Human H-FABP, and a reagent therefor and the like, the cardiotoxicity can be detected sensitively at an early stage, which enables physicians to conduct medical procedures at an early stage of cardiotoxicity expression, such as change of pharmaceutical agents and the like.

This application is based on application No. 2002-093688 filed in Japan, the contents of which are incorporated hereinto by reference.

**Claims**

1. A method for determining toxicity to the heart of an anthracycline-type anticancer chemotherapeutic agent, which comprises detecting human H-FABP in the blood separated from human.

2. The method of claim 1, wherein the detection of human H-FABP is performed by an immunochemical method using an antibody that recognizes human H-FABP.

3. The method of claim 2, wherein the immunochemical method is an enzyme immunochemical method, a latex agglutination assay or an immunochromatographic assay.

4. The method of claim 2, wherein the antibody is a monoclonal antibody.

5. The method of claim 1, wherein the anthracycline-type anticancer chemotherapeutic agent is adriamycin or daunorubicin hydrochloride.

6. A reagent for determining toxicity to the heart of an anthracycline-type anticancer chemotherapeutic agent, which is used for performing the method of any of claims 1 to 5.

**7.** A reagent for determining toxicity to the heart of an anthracycline-type anticancer chemotherapeutic agent, which comprises an antibody that recognizes human H-FABP.

**8.** The reagent of claim 7, wherein the antibody is a monoclonal antibody.

**9.** The reagent of claim 7, wherein the anthracycline-type anticancer chemotherapeutic agent is adriamycin or daunorubicin hydrochloride.

**10.** A commercial package comprising the reagent of any of claims 7 to 9, and a written matter associated therewith, the written matter stating that said reagent can or should be used for determining toxicity to the heart of an anthracycline-type anticancer chemotherapeutic agent.

**11.** A kit for determining toxicity to the heart of an anthracycline-type anticancer chemotherapeutic agent, which comprises an antibody that recognizes human H-FABP.

**12.** The kit of claim 11, wherein the antibody is a monoclonal antibody.

**13.** The kit of claim 11, wherein the anthracycline-type anticancer chemotherapeutic agent is adriamycin or daunorubicin hydrochloride.

**14.** Use of an antibody that recognizes human H-FABP for determining toxicity to the heart of an anthracycline-type anticancer chemotherapeutic agent.

**15.** The use of claim 14, which comprises detecting human H-FABP in the blood separated from human.

**16.** The use of claim 15, wherein the detection of human H-FABP is performed by an enzyme immunochemical method, a latex agglutination assay or an immunochromatographic assay.

**17.** The use of claim 14, wherein the antibody is a monoclonal antibody.

**18.** The use of claim 14, wherein the anthracycline-type anticancer chemotherapeutic agent is adriamycin or daunorubicin hydrochloride.

## INTERNATIONAL SEARCH REPORT

International application No.
PCT/JP03/03924

**A. CLASSIFICATION OF SUBJECT MATTER**

Int.Cl$^7$ G01N33/68, G01N33/53, G01N33/15

According to International Patent Classification (IPC) or to both national classification and IPC

**B. FIELDS SEARCHED**

Minimum documentation searched (classification system followed by classification symbols)

Int.Cl$^7$ G01N33/68, G01N33/53, G01N33/15

Documentation searched other than minimum documentation to the extent that such documents are included in the fields searched

| | | | |
|---|---|---|---|
| Jitsuyo Shinan Koho | 1922-1996 | Toroku Jitsuyo Shinan Koho | 1994-2003 |
| Kokai Jitsuyo Shinan Koho | 1971-2003 | Jitsuyo Shinan Toroku Koho | 1996-2003 |

Electronic data base consulted during the international search (name of data base and, where practicable, search terms used)

BIOSIS(DIALOG), WPI(DIALOG)

**C. DOCUMENTS CONSIDERED TO BE RELEVANT**

| Category* | Citation of document, with indication, where appropriate, of the relevant passages | Relevant to claim No. |
|---|---|---|
| Y | IBRAHIM IA. et al., "Depression of cytosolic fatty acid binding protein(H-FABP) expression in adriamycin-induced cardiomyopathy and its reversal by L-carnitine.", FASEB Journal 13(5 PART 2): pA907, 15 March, 1999 (15.03.99), | 1-18 |
| Y | JP 2001-037486 A (Tanabe Seiyaku Co., Ltd.), 13 February, 2001 (13.02.01), Par. Nos. [0025] to [0027] (Family: none) | 1-18 |
| Y | JP 04-031762 A (Dainippon Pharmaceutical Co., Ltd.), 03 February, 1992 (03.02.92), Full text (Family: none) | 1-18 |

☐ Further documents are listed in the continuation of Box C. ☐ See patent family annex.

* Special categories of cited documents:
- "A" document defining the general state of the art which is not considered to be of particular relevance
- "E" earlier document but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| | |
|---|---|
| Date of the actual completion of the international search<br>21 April, 2003 (21.04.03) | Date of mailing of the international search report<br>06 May, 2003 (06.05.03) |
| Name and mailing address of the ISA/<br>Japanese Patent Office | Authorized officer |
| Facsimile No. | Telephone No. |

Form PCT/ISA/210 (second sheet) (July 1998)